(19) **Europäisches Patentamt**

**European Patent Office**

**Office européen des brevets**

(11) **EP 1 020 184 A1**

(12) **EUROPEAN PATENT APPLICATION**

(43) Date of publication:
**19.07.2000 Bulletin 2000/29**

(51) Int. Cl.7: **A61K 31/135**, A61K 9/20

(21) Application number: **00300304.3**

(22) Date of filing: **17.01.2000**

| | |
|---|---|
| (84) Designated Contracting States:<br>**AT BE CH CY DE DK ES FI FR GB GR IE IT LI LU MC NL PT SE**<br>Designated Extension States:<br>**AL LT LV MK RO SI** | (72) Inventor:<br>**SHERMAN, Bernard Charles**<br>**Willowdale, Ontario M2L 2K1 (CA)** |
| (30) Priority: **18.01.1999 CA 2259730** | (74) Representative:<br>**Votier, Sidney David**<br>**CARPMAELS & RANSFORD**<br>**43, Bloomsbury Square**<br>**London WC1A 2RA (GB)** |
| (71) Applicant:<br>**SHERMAN, Bernard Charles**<br>**Willowdale, Ontario M2L 2K1 (CA)** | |

(54) **Sustained release tablets containing bupropion hydrochloride**

(57)    A sustained release tablet comprising bupropion hydrochloride and hydroxyethylcellulose.

**EP 1 020 184 A1**

Printed by Xerox (UK) Business Services
2.16.7 (HRS)/3.6

**Description**

BACKGROUND OF THE INVENTION

Bupropion hydrochloride is a well known antidepressant. It is sold in the United States by Glaxo-Wellcome Inc. as prompt release tablets under the tradename WELLBUTRIN® and sustained release tablets under the tradename WELLBUTRIN SR®.

Bupropion hydrochloride is known to be relatively unstable, such that tablets containing bupropion hydrochloride will degrade at an unacceptably high rate unless the tablets are made by a method or using ingredients which result in improved stability. U.S. patent 5358970 discloses stabilization of bupropion hydrochloride by including in the tablets a stabilizer selected from a specified group of acids.

U.S. patent 5427798 discloses that bupropion hydrochloride is advantageously administered as sustained release tablets and certain formulations for making such tablets are disclosed and claimed. The formulations disclosed and claimed all comprise hydroxypropyl methylcellulose as the agent for controlling the drug release rate. Wellbutrin SR® tablets sold in the United States appear to be made within the scope of the claims of U.S. patent 5427798.

U.S. patent 5427798 discloses that the dissolution rate of bupropion hydrochloride from the sustained release tablets disclosed therein is preferably as follows:

i) Between about 20% and about 60% in 1 hour,
ii) Between about 50% and about 95% in 4 hours, and
iii) Not less than about 75% in 8 hours.

The test conditions and apparatus used to determine the dissolution rate are specified to be:

Apparatus: USP Rotating Paddle (Apparatus II)
Stirring Rate: 50 rpm
Medium: 900 mL of distilled water
Temperature: 37°C

All of the tablets in the examples in U.S. patent 5427798 comprise glycine hydrochloride as acidic stabilizer, in addition to the bupropion hydrochloride and hydroxypropyl methylcellulose; and in each example the process of manufacture includes the steps of dissolving the acidic stabilizer in water and using the resultant solution to wet-granulate a mixture of hydroxypropyl methylcellulose and microcrystalline cellulose.

Such a process has the disadvantage of requiring the use of water and requiring the steps of preparing the solution, using the solution to wet-granulate a mixture of powders, and drying the wet mass.

The object of the present invention is to enable production of stable sustained release tablets comprising bupropion hydrochloride, which do not require use of hydroxypropyl methylcellulose and can be made without requiring a wet-granulation process.

DESCRIPTION OF THE INVENTION

It has been found that inclusion of hydroxyethylcellulose in tablets comprising bupropion hydrochloride, along with an acidic stabilizer, enables the production of stable sustained release tablets having appropriate dissolution rate, and that such tablets can be made without requiring a wet-granulation process.

Hydroxyethylcellulose (hereinafter referred to "HEC") is sold in the United States and elsewhere under the tradename Natrosol®. HEC is a hydroxyethyl ether of cellulose. The cellulose molecule is comprised of a chain of anhydroglucose units, each of which contains three hydroxyls capable of reaction. Hydroxyethyl groups can be introduced into the cellulose molecule by reacting with ethylene oxide at the hydroxyls in the cellulose chain. Further hydroxyethyl groups can be introduced by reacting with ethylene oxide at the hydroxyls at the end of hydroxyethyl groups that have previously been added.

The average number of moles of ethylene oxide that become attached to each anhydroglucose unit in cellulose in these two ways is called "moles of substituent combined" or "MS".

A preferred HEC is that having MS of 2.5. This is available under the tradename Natrosol 250®. Viscosity of a solution of HEC in water increases with increased molecule weight of the HEC. Natrosol 250® is available in various viscosity types as follows:

| | Viscosity in cps at 25°C at various concentrations in water | | |
|---|---|---|---|
| Viscosity Type | 1% | 2% | 5% |
| 250HH | 3400-5000 | | |
| 250H4 | 2600-3300 | | |
| 250H | 1500-2500 | | |
| 250MH | 800-1500 | | |
| 250M | | 4500-6500 | |
| 250K | | 1500-2500 | |
| 250G | | 150-400 | |
| 250E | | 25-105 | |
| 250J | | | 150-400 |
| 250L | | | 75-150 |

Preferred viscosity types are those with viscosity of above about 800 cps in 1 % aqueous solution. Most preferred is the type with viscosity of about 1500 to 2500 cps in 1% aqueous solution, which is available as Natrosol 250H®.

The amount of HEC per tablet will preferably be from about 10 to about 60 parts per part of bupropion hydrochloride by weight. The amount of HEC per tablet will be more preferably from about 20 to about 40 parts per part of bupropion hydrochloride by weight.

In addition to comprising bupropion hydrochloride and HEC, the sustained release tablets will preferably also comprise another ingredient as a binder to increase the hardness of the tablets when the mixture of ingredients is compressed into tablets on a tablet press. This binder will preferably be either microcrystalline cellulose or methylcellulose.

Methylcellulose is available in the United States and elsewhere under the tradename Methocel A® Methocel A® is available in several different viscosity grades corresponding to different molecular weights.

The lowest available viscosity grade, corresponding to the lowest molecular weight, is sold as Methocel A15LV® which has a viscosity of 15 cps in 2% aqueous solution at 25°C. This lowest viscosity grade is preferred for use in the present invention.

The tablets of the present invention will preferably also comprise an acidic stabilizer, which will preferably be sodium bisulfate.

The tablets will preferably also comprise a lubricant, such as, for example, magnesium stearate to avoid sticking to the tooling in the tabletting process.

The tablets will optionally also comprise a glidant such as, for example, colloidal silicon dioxide.

The tablets will be made by mixing the ingredients and compressing the mixture into tablets on a tablet press as it has surprisingly been found that the use HEC enables the obtention of a tablet exhibiting adequate hardness and slower-release properties without the need to wet-granulate the mixture. Preferably the mixture will be made by a simple dry mix process; that is to say, simply by mixing ingredients together in dry form, without the addition of water or another solvent and the subsequent removal of such solvent by drying.

If the dry mixture of ingredients does not flow well enough for direct tabletting, then the mixture can be slugged or compacted and then ground up into granules. The granules can then be compressed into tablets with or without the addition of other ingredients.

After the tablets are made they can optionally be coated with a film coating.

The tablets of the present invention will preferably have the same dissolution profile as specified in U.S. patent 5427798. That is to say;

i) Between about 20% and about 60% in 1 hour,
ii) between about 50% and about 95% in 4 hours, and
iii) not less than about 75% in 8 hours.

The test conditions and apparatus used to determine dissolution rate are:

| Apparatus | USP Rotating Paddle (Apparatus II) |
|---|---|
| Stirring Rate | 50 rpm |
| Medium | 900 mL of distilled water |
| Temperature | 37°C |

The following example is representative of the invention, but not limiting.

Ingredients were mixed in the relative amounts as follows:

Example 1

| Bupropion Hydrochloride | 100.0 |
|---|---|
| Natrosol 250H | 32.0 |
| Methocel A15LV | 80.0 |
| Sodium Bisulfate | 5.0 |
| Magnesium Stearate | 2.2 |
| Colloidal Silicon Dioxide | 0.8 |
| | 220.0 |

The ingredients were mixed, the mixture was compacted, the compacted material was ground up into granules, and the granules were recompressed on a tablet press into tablets of net weight 220 mg each.

Each tablet thus contained 100 mg of bupropion hydrochloride. Sample tablets were tested for dissolution rate by the aforesaid method and the results were found to comply with the aforesaid specifications.

**Claims**

1.  A sustained release tablet comprising bupropion hydrochloride and hydroxyethylcellulose for which the dissolution profile complies with specifications as follows:

    i) Between about 20% and about 60% in 1 hour,
    ii) Between about 50% and about 95% in 4 hours, and
    iii) Not less than about 75% in 8 hours.

    when tested using USP Rotating Paddle Apparatus, at a stirring rate of 50 rpm, using as medium 900 mL of distilled water at 37°C.

2.  A tablet of claim 1 wherein the amount of hydroxyethylcellulose per tablet is from about 10 to about 60 parts per part of bupropion hydrochloride by weight.

3.  A tablet of claim 1 wherein the amount of hydroxyethylcellulose per tablet in from about 20 to about 40 parts per part of bupropion hydrochloride by weight.

4.  A tablet according to Claim 2 or 3 wherein hydroxyethylcellulose has a viscosity of at least about 800 cps in 1% aqueous solution at 25°C.

**5.** A tablet according to Claim 2 or 3 wherein hydroxethycellulose has a viscosity of from about 1500 cps to about 2500 cps in 1% aqueous solution at 25°C.

**6.** A tablet of any of claims 1 to 5 further comprising a binder.

**7.** A tablet of claim 6 wherein the binder is microcrystalline cellulose.

**8.** A tablet of claim 6 wherein the binder is methylcellulose.

**9.** A tablet of any of claims 1 to 8 further comprising an acidic stabilizer.

**10.** A tablet of claim 9 wherein the acidic stabilizer is sodium bisulfate.

**11.** A process of making a tablet of any of claims 1 to 10 which comprises the steps of mixing in dry form and compression into tablets, without the use of water or any other liquid for wet granulation.

**12.** A tablet of any of claims 1 to 10 when made by a process of claim 11.

**European Patent Office**

**EUROPEAN SEARCH REPORT**

**Application Number**

EP 00 30 0304

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 98 47491 A (ODIDI, ISA, ET AL.) 29 October 1998 (1998-10-29) * claims 1,4,13,28 * * page 9, line 18 - line 24 * * page 16; example 7 * --- | 1,6,7 | A61K31/135 A61K9/20 |
| P,X | WO 99 07342 A (ALZA CORPORATION) 18 February 1999 (1999-02-18) * claims 1-6 * * page 9, line 8 - line 15 * * page 16, line 14 - page 17, line 3 * * page 18, line 15 - line 27 * ----- | 1-9 | |

|  |  |  | TECHNICAL FIELDS SEARCHED (Int.Cl.7) |
|---|---|---|---|
|  |  |  | A61K |

The present search report has been drawn up for all claims

| Place of search | Date of completion of the search | Examiner |
|---|---|---|
| THE HAGUE | 25 April 2000 | Ventura Amat, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT
## ON EUROPEAN PATENT APPLICATION NO.

EP 00 30 0304

This annex lists the patent family members relating to the patent documents cited in the above-mentioned European search report.
The members are as contained in the European Patent Office EDP file on
The European Patent Office is in no way liable for these particulars which are merely given for the purpose of information.

25-04-2000

| Patent document cited in search report | Publication date | Patent family member(s) | | Publication date |
|---|---|---|---|---|
| WO 9847491 A | 29-10-1998 | AU | 6817098 A | 13-11-1998 |
| | | CA | 2216215 A | 05-10-1998 |
| WO 9907342 A | 18-02-1999 | AU | 8699298 A | 01-03-1999 |

EPO FORM P0459